# DEMANDE DE BREVET EUROPEEN

(11) **EP 0 541 418 A1**
(43) Date de publication de la demande: **12.05.1993**
(21) Numéro de dépôt: 92402883.0
(22) Date de dépôt: 22.10.1992
(51) Int. Cl.: C12N 7/02, C12N 7/04, C12N 15/33, A61K 39/02

(54) **Préparation d'antigènes et de vaccins du virus de la mystery disease, antigènes et vaccins obtenus pour la prévention de cette maladie**

(30) Priorité: 29.10.1991 FR 9113338
(71) Demandeur: RHONE MERIEUX S.A., 69002 Lyon (FR)
(72) Inventeur: Brun, André, F-69300 Caluire (FR); Vaganay, Alain, F-69100 Villeurbanne (FR); Tardy, Marie-Claude, F-69003 Lyon (FR); Vandeputte, Joris, F-38790 Diemoz (FR)
(74) Mandataire: Bernasconi, Jean

(57) **Abrégé**

Le procédé d'isolement du virus de la Mystery Disease comportent le prélèvement d'organes de porcs malades ou infectés, le broyage du prélèvement et des passages du surnageant de broyage sur des cellules sensibles hétérologues ou homologues, puis la récolte du surnageant. Pour la production industrielle du virus de la Mystery Disease, on cultive le virus sur des cellules sensibles hétérologues ou homologues.

Les préparations d'antigène viral purifié de la Mystery Disease comprennent des particules virales vivantes, atténuées ou non, ou des particules inactivées ou des antigènes de sous-unités ou des antigènes exprimés par recombinaison génétique à partir de gènes du virus isolé. Vaccins contenant une quantité vaccinante d'un tel antigène.

## Description

Préparation d'antigènes et de vaccins du virus de la Mystery Disease, antigènes et vaccins obtenus pour la prévention de cette maladie.

La présente invention a trait à la préparation d'antigènes et de vaccins du virus de la Mistery Disease, ainsi qu'aux antigènes et aux vaccins obtenus.

La maladie dite Mystery Disease (M.D.) ou encore Porcine Reproductive Respiratory Syndrome (P.R.R.S.) a commencé à être individualisée, chez le porc, en 1986 aux Etats-Unis et en 1990 en Europe. Cette maladie se traduit essentiellement chez le porc, par des signes d'abattement, une anorexie, et une hyperthermie de l'ordre de 40° C, que l'on observe classiquement sur les truies dans les élevages atteints par la maladie. Ces signes sont accompagnés ou suivis de troubles de la reproduction (mises bas précoces ou tardives et naissance de porcelets mort-nés, momifiés ou chétifs et retours en chaleurs des truies). Un syndrome respiratoire peut être observé chez les porcelets avec des lésions de pneumonie interstitielle. Des porcs plus âgés peuvent également être atteints de troubles respiratoires. Toute cette symptomatologie peut être accompagnée de maladies provoquées par des infections occasionnelles classiquement observées chez le porc.

Les inventeurs ont réussi à isoler et à identifier un nouveau virus, responsable de cette maladie. Ce virus est de type Myxovirus, selon l'analyse réalisée au microscope électronique, et a comme caractéristique de ne pas être neutralisé par des sérums antigrippe porcine H1N1 et H3N2.

Une souche de ce virus identifiée sous la désignation P129-294 a été déposée à la Collection Nationale de Cultures de Micro-organismes tenue à l'Institut Pasteur sous le n° I-1153.

Par ailleurs, il s'est ensuite avéré que ce virus présente un certain nombre de traits du virus de la maladie de Newcastle, tout en étant différent des souches connues de ce virus.

La présente invention a pour objet un procédé d'isolement du virus et son utilisation pour la préparation d'antigènes.

Un tel procédé comporte le prélèvement d'organes d'animaux malades ou infectés, le broyage du prélèvement, et des passages du surnageant sur des cellules sensibles hétérologues ou homologues telles que, notamment, les cellules de la lignée Vero, MDCK (en présence de trypsine), ST, BHK ou encore sur oeufs embryonnés.

On préfère effectuer le prélèvement à partir de poumon d'animal infecté ou encore d'un pool d'organes comprenant, par exemple, coeur, rate, foie, rein et tissus lymphoïdes.

Les récoltes ne sont pas neutralisées par des sérums antigrippe porcine H1N1 et H3N2. Les tests d'hémagglutination sont positifs à partir de l'antigène produit sur oeufs embryonnés.

L'invention a également pour objet un procédé de production industrielle de ce virus dans lequel on cultive le virus sur des cellules sensibles hétérologues ou homologues telles que notamment les cellules de la lignée Vero, MDCK (en présence de trypsine), ST, BHK ou encore sur des oeufs embryonnés.

Le virus récolté peut être utilisé pour la préparation d'antigènes. A cette fin, il est, de préférence, convenablement purifié selon des procédures usuelles pour les Myxovirus, par exemple l'ultra-centrifugation ou la chromatographie. Il peut également être concentré par les techniques usuelles.

Selon l'utilisation envisagée, les préparations d'antigènes selon l'invention peuvent être constituées de particules virales vivantes, atténuées ou non, de particules inactivées, d'antigènes de sous-unités ou encore d'antigènes obtenus par recombinaison génétique à partir de gènes du virus isolé, insérés pour être exprimés dans le génome d'hôtes recombinants procaryotes ou eucaryotes.

L'invention a également pour objet les vaccins réalisés à partir des antigènes précités, contenant une quantité vaccinante efficace d'antigènes, dans des véhicules convenables.

Ces vaccins peuvent être prévus pour être administrés aux porcs mais également à d'autres animaux qui pourraient s'avérer sensibles à ce virus.

Un vaccin atténué peut être préparé par passages du virus sur culture cellulaire.

La quantité de virus par dose vaccinale est de préférence comprise entre 10³ et 10⁸ DICC₅₀ par dose.

Le vaccin atténué vivant peut être présenté sous forme liquide ou lyophilisée en présence de stabilisateurs de formulations très variées, pouvant inclure des sucres, des protéines et des tampons. Le vaccin peut être adjuvé à l'aide d'adjuvants minéraux ou organiques.

Le vaccin vivant peut être administré à des animaux pour les protéger dès le début de la période d'engraissement ou avant l'insémination artificielle ou la saillie, ou au cours de la gestation, en une et de préférence en deux injections, à trois ou quatre semaines d'intervalle.

Un vaccin inactivé peut être préparé à partir de suspensions virales obtenues par passages sur des systèmes cellulaires homologues (porcin) ou hétérologues, puis inactivation par des agents inactivants chimiques usuels tels que la Beta-propiolactone, des enzymes ou des solvants organiques ou des détergents. L'inactivation peut également être obtenue par action physique tels que rayonnement ultra-violet, irradiations gamma ou rayons X. L'agent inactivant peut être neutralisé si nécessaire.

Le vaccin inactivé contient de préférence au moins l'équivalent de 10⁵ DICC₅₀ par dose vaccinale, concentration déterminée avant inactivation.

Le vaccin inactivé peut être administré aux animaux pour les protéger dès le début de la période d'engraissement, ou avant l'insémination artificielle ou la saillie, ou en cours de gestation, en une et de préférence deux injections à trois ou quatre semaines d'intervalle. On préfère que le vaccin contienne un adjuvant d'origine minérale ou organique.

Un vaccin recombinant peut être obtenu, par exemple, par insertion de la séquence codant pour l'antigène recherché du virus dans le génome de l'hôte. L'hôte peut être un virus, notamment pour la réalisation d'un vaccin vivant.

Cet hôte peut également être un système bactérien, de levure, ou d'autres cellules eucaryotes. Dans ce cas, l'hôte est de préférence utilisé pour la production d'antigènes qui sont ensuite purifiés et conditionnés pour faire le vaccin.

Le vaccin peut être présenté sous forme monovalente ou associé à d'autres agents viraux ou bactériens responsables de maladies chez le porc.

L'invention a également pour objet, à titre de vaccin équivalent contre la Mystery Disease du porc, un vaccin caractérisé en ce qu'il comporte une quantité thérapeutiquement efficace chez le porc, d'une souche virulente de virus de Newcastle, convenablement inactivée, ou d'antigènes vaccinants d'une telle souche, avec, de préférence, un adjuvant classique.

D'autres avantages et caractéristiques de l'invention apparaîtront à la lecture de la description suivante, faite à titre d'exemple non limitatif.

### Exemple 1 : Isolement de la souche virale

Des prélèvements d'organes ont été réalisés sur une truie provenant d'un élevage de porcs d'Allemagne et identifiée sous le n° 294.

Les prélèvements provenaient du coeur, de la rate, du foie, du rein, du tissu lymphoïde et du poumon, exploités sans congélation.

### a) Méthode :

Broyats d'organes : chaque prélèvement est broyé individuellement dans milieu MEM + antibiotiques.

Dilution : poids organe par volume d'environ 1 pour 10.

Centrifugation clarifiante pour récupération du liquide surnageant (LS).

Filtration à 0,22 µ.

### b) Essais sur cultures cellulaires.

Premiers passages
A partir de :
1) LS de broyat de poumon
2) LS pool d'organes : coeur, rate, foie, rein et tissu lymphoïde
   . cellules utilisées : lignées Vero, IRO.3,ST, MDCK (en présence de trypsine), BHK, cellules primaires de rein de porc
   . inoculation sur des cultures en couche établie (Falcons de 25 cm²)
   . observation des cellules et congélation à -70°C.

Deuxièmes passages
A partir des premiers passages décongelés.

Sur même type de cellules que les premiers passages ; les cellules sont utilisées en inoculation simultanée.

Observation et congélation.

### c) Essais sur oeufs embryonnés

. Inoculation sur oeufs SPF embryonnés de 9 jours
. Inoculum : LS broyat de poumon de la truie 294. 0,1 ml d'inoculum non dilué ou dilué au 1/10 dans PBS + antibiotiques est inoculé par voie intra-allantoïdienne.
. Après 3 jours d'incubation, les oeufs sont vidés et le liquide allantoïdien est récolté (récolte individuelle de chaque oeuf).
. Un test d'hémagglutination vis-à-vis de globules rouges de poule (concentration 20.10⁶ globules rouges par ml) est pratiqué sur tous les échantillons récoltés.
. Les passages successifs sont réalisés dans les conditions décrites ci-dessus.

### d) Résultats

1) Résultats des cultures cellulaires
   Sur deux types cellulaires, des altérations de la couche cellulaire ont pu être notées :
   - sur cellules Vero : les cellules des Falcons inoculés présentent, lors du second passage, un aspect du tapis cellulaire différent des cellules du Falcon témoin. Cet aspect anormal est noté à +7 jours après inoculation.
   - sur cellules MDCK en présence de trypsine (concentration 20 µg/ml) : les cellules inoculées par le pool d'organes montrent un aspect différent des cellules témoins à +6 jours de culture.
2) Résultats sur oeufs
   Premier passage : aucune hémagglutination n'a pu être révélée.
   Deuxième passage : 1 oeuf présente une hémagglutination. Le titre d'hémagglutination HA est de 640.
   Troisième passage :
   . titre HA pouvant atteindre 1024
   . présence d'HA non neutralisée par des sérums antigrippe porcine H1N1 et H3N2

   Quatrième passage : hémagglutination confirmée avec des titres pouvant atteindre 2048.

En outre, une mortalité importante des oeufs a pu être notée aux deuxième, troisième et quatrième passages, pouvant dépasser 50% des oeufs inoculés.

Un test d'hémagglutination positif a été réalisé vis-à-vis de globules rouges de cobayes. A l'aide de l'antigène hémagglutinant du 3ème passage, des cellules de lignée ST, Vero et des cellules primaires de rein de porc SPF ont été inoculées, un effet cytopathogène a été observé sur ces 3 cellules, effet cytopathogène plus marqué que sur les cellules inoculées directement avec le L.S. de poumon. L'effet hémagglutinant avait disparu après passages sur cellules.

A partir de la suspension virale du 3ème passage sur oeufs, 2 porcs d'un poids de 30 kg ont été inoculés par voie intraveineuse sous un volume de 1 cc. Ces 2 porcs ont présenté des signes d'abattement, d'anorexie et d'hyperthermie de l'ordre de 40°C durant 7 jours au maximum. Ces symptômes sont ceux classiquement observés sur les truies dans les élevages atteints de P.R.R.S. ou M.D.

L'agent isolé présente donc les caractéristiques suivantes :
- il provoque l'hémagglutination des globules rouges de cobayes à partir d'antigène multiplié sur oeufs embryonnés,
- il n'est pas inhibé par des sérums antigrippe porcine,
- il produit un effet cytopathogène sur différentes cellules,
- ses propriétés visualisées en microscopie électronique l'apparentent au groupe des Myxovirus.

Ces propriétés sont également celles de la souche P129-294 déposée à la CNCM.

### Exemple 2 : Préparation d'un vaccin vivant

Le vaccin vivant est préparé à partir de la souche P129-294. La souche est multipliée par passages sur cellules Vero. La récolte s'effectue après. Le surnageant récolté, traité, est lyophilisé avec un stabil isateur SPGA.

Le vaccin est conditionné en doses de 10, 50 et 100.

### Exemple 3 : Préparation d'un vaccin inactivé

Le virus de la souche P129-294 est multiplié sur cellules ST. A la suite du cinquième passage, le surnageant contenant le virus est récolté, concentré et purifié. Il est inactivé par la Beta-propiolactone, puis adjuvé sur hydroxyde d'aluminium.

Le vaccin est conditionné en doses de 10 et 50.

## Revendications

1. Procédé d'isolement du virus de la Mystery Disease comportant le prélèvement d'organes de porcs malades ou infectés, le broyage du prélèvement et des passages du surnageant de broyage sur des cellules sensibles hétérologues ou homologues, puis la récolte du surnageant.

2. Procédé selon la revendication 1, caractérisé en ce que les passages sont effectués sur cellules de la lignée Vero, MDCK, ST, BHK ou sur oeufs embryonnés.

3. Procédé de production industrielle du virus de la Mystery Disease dans lequel on cultive le virus sur des cellules sensibles hétérologues ou homologues.

4. Procédé selon la revendication 3, caractérisé en ce que l'on cultive le virus sur des cellules de la lignée Vero, MDCK, ST, BHK et l'on récolte le dernier surnageant.

5. Procédé selon la revendication 3, caractérisé en ce que l'on cultive le virus sur oeufs embryonnés et on récupère le liquide allantoïdien.

6. Préparation d'antigène viral purifié de la Mystery Disease comprenant des particules virales vivantes, atténuées ou non, ou des particules inactivées ou des antigènes de sous-unités ou des antigènes exprimés par recombinaison génétique à partir de gènes du virus isolé.

7. Préparation d'antigène de la souche virale P 129-294 déposée à la CNCM sous le n° I-1153.

8. Vaccin contre la Mystery Disease, caractérisé en ce qu'il contient, dans un véhicule approprié, une quantité vaccinante d'un antigène selon l'une des revendications 5 et 6.

9. Vaccin atténué selon la revendication 8, caractérisé en ce qu'il est préparé par passages du virus sur culture cellulaire.

10. Vaccin atténué selon la revendication 9, caractérisé en ce que la quantité de virus par dose vaccinale est comprise entre 10³ et 10⁸ DICC₅₀.

11. Vaccin inactivé selon la revendication 8, caractérisé en ce que le surnageant de culture est inactivé par un agent inactivant chimique ou physique.

12. Vaccin inactivé selon la revendication 11, caractérisé en ce qu'il contient au moins l'équivalent de 10⁵ DICC₅₀ par dose vaccinale.

13. Vaccin contre la Mystery Disease, caractérisé en ce qu'il contient une dose thérapeutiquement efficace d'une souche virulente de virus de Newcastle ou d'antigènes vaccinants d'une telle souche.
